(19) Europäisches Patentamt
European Patent Office
Office européen des brevets

(11) Veröffentlichungsnummer: **0 019 081**
**B1**

# (12) EUROPÄISCHE PATENTSCHRIFT

(45) Veröffentlichungstag der Patentschrift: **24.10.84**

(51) Int. Cl.³: **C 07 D 311/30**, A 61 K 31/35

(21) Anmeldenummer: **80101894.6**

(22) Anmeldetag: **09.04.80**

(54) **Neue Flavonderivate, deren Herstellung, diese neuen und bekannte Flavone als antivirale Wirkstoffe und sie enthaltende pharmazeutische Präparate.**

(30) Priorität: **10.04.79 GB 7912610**
**25.02.80 GB 8006259**

(43) Veröffentlichungstag der Anmeldung: **26.11.80 Patentblatt 80/24**

(45) Bekanntmachung des Hinweises auf die Patenterteilung: **24.10.84 Patentblatt 84/43**

(84) Benannte Vertragsstaaten: **BE CH DE FR GB IT LI LU NL SE**

(56) Entgegenhaltungen:
**EP - A - 0 004 579**
**AT - B - 306 716**
**DE - A - 1 493 546**
**DE - A - 1 793 025**
**DE - A - 2 427 597**

**Steinegger, Hänsel: Lehrbuch d.allgem. Pharmakognosie, Berlin 1963, S. 168-169**

**Die Akte enthält technische Angaben, die nach dem Eingang der Anmeldung eingereicht wurden und die nicht in dieser Patentschrift enthalten sind.**

(73) Patentinhaber: **F. HOFFMANN-LA ROCHE & CO. Aktiengesellschaft, CH-4002 Basel (CH)**

(72) Erfinder: **Ishitsuka, Hideo, 1-go, 405 Katsura-cho 1-banchi 1, Totsuka-ku Yokohama-shi, Kanagawa-ken (JP)**
Erfinder: **Shirai, Haruyoshi, 1622-4, Takamori, Isehara-shi, Kanagawa-ken (JP)**
Erfinder: **Umeda, Isao 204 Height Hakuraku, 4-4-20, Rokkakubashi Kanagawa-ku, Yokohama-shi, Kanagawa-ken (JP)**
Erfinder: **Suhara, Yasuji 9-506 Dream Height, Matanocho 1403-banchi Totsuka-ku, Yokohama-shi, Kanagawa-ken (JP)**

(74) Vertreter: **Lederer, Franz, Dr. et al, Patentanwälte Dr. Franz Lederer Reiner F. Meyer Lucile-Grahn-Strasse 22, D-8000 München 80 (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



ACTORUM AG

**Beschreibung**

Die vorliegende Erfindung betrifft neue 3-Alkoxyflavonderivate, ein Verfahren zu deren Herstellung und pharmazeutische Präparate mit antiviraler Aktivität auf der Basis von 3-Alkoxyflavonderivaten.

Die vorliegende Erfindung betrifft insbesondere antivirale Mittel, die als Wirksubstanz ein 3-Alkoxyflavonderivat der allgemeinen Formel

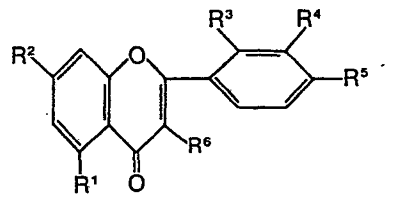
I

worin

$R^1$ Hydroxy, $C_{1-4}$-Alkoxy, $C_{2-7}$-Alkanoyloxy, $C_{2-7}$-Alkoxycarbonyloxy oder Nicotinoyloxy;

$R^2$ Hydroxy oder $C_{1-4}$-Alkoxy;

$R^3$ Wasserstoff oder $C_{1-4}$-Alkoxy

$R^4$ Wasserstoff, Hydroxy oder $C_{1-4}$-Alkoxy;

$R^5$ Hydroxy, $C_{1-4}$-Alkoxy, $C_{2-7}$-Alkanoyloxy, L-Lysyloxy, L-Alanyloxy, L-Glutaminyloxy, α-Glutamyloxy, Glucosyloxy, Mannosyloxy, Galaktosyloxy, einen 1-Hydroxy-1,2-ethandicarbonsäurerest, einen 1,2-Dihydroxy-1,2-ethandicarbonsäurerest, Amino, Nicotinoyloxy oder $C_{2-7}$-Alkoxycarbonyloxy und

$R^6$ $C_{1-4}$-Alkoxy darstellen,

enthalten.

Die vorliegende Erfindung betrifft ebenfalls neue Verbindungen der allgemeinen Formel

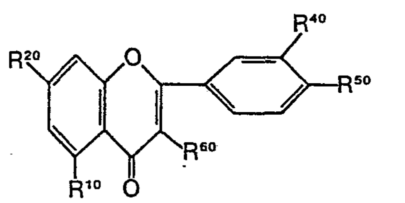
II

worin

$R^{10}$ Hydroxy, $C_{2-7}$-Alkanoyloxy, $C_{2-7}$-Alkoxycarbonyloxy oder Nicotinoyloxy;

$R^{20}$ Hydroxy oder $C_{1-4}$-Alkoxy;

$R^{40}$ Wasserstoff oder $C_{1-4}$-Alkoxy;

$R^{50}$ Hydroxy, $C_{2-7}$-Alkanoyloxy, L-Lysyloxy, L-Alanyloxy, L-Glutaminyloxy, α-Glutamyloxy, Glucosyloxy, Mannosyloxy, Galaktosyloxy, einen 1-Hydroxy-1,2-ethandicarbonsäurerest, einen 1,2-Dihydroxy-1,2-ethandicarbonsäurerest, Amino, Nicotinoyloxy oder $C_{2-7}$-Alkoxycarbonyloxy und

$R^{60}$ $C_{1-5}$-Alkoxy darstellen,

mit der Einschränkung, dass $R^{50}$ nicht Acetoxy darstellt, wenn $R^{10}$ Acetoxy ist, und dass $R^{60}$ nicht Methoxy darstellt, wenn $R^{50}$ Hydroxy ist,

sowie ein Verfahren zu deren Herstellung.

Bevorzugte $C_{1-4}$-Alkoxygruppen sind Methoxy und Äthoxy. Beispiele von $C_{2-4}$-Alkanoyloxygruppen sind Acetoxy, Propionyloxy, Butyryloxy, Isobutyryloxy und Pivaloyloxy.

Der Stand der Technik wird illustriert durch die im Lehrbuch der allgemeinen Pharmakognosie Berlin 1963, Seiten 168–169 und in der Deutschen Offenlegungsschrift No. 2 427 597 beschriebenen Flavone mit pharmazeutischer Verwendung, die sich jedoch strukturell von den erfindungsgemässen 3-Alkoxyflavonen unterscheiden.

Repräsentative Beispiele von Verbindungen der Formel I sind:

A. Neue Verbindungen

4′-Acetoxy-5-hydroxy-3,3′,7-trimethoxyflavon;

5-Hydroxy-4′-(L-lysyloxy)-3,3′,7-trimethoxyflavon;

4′-(β-D-Glucopyranosyloxy)-5-hydroxy-3,3′,7-trimethoxyflavon;

4′-(L-Alanyloxy)-5-hydroxy-3,3′,7-trimethoxyflavon;

4′-(L-Glutaminyloxy)-5-hydroxy-3,3′,7-trimethoxyflavon;

4′-(1-α-Glutamyloxy)-5-hydroxy-3,3′,7-trimethoxyflavon;

4′-(3-Carboxy-2,3-dihydroxypropionyloxy)-5-hydroxy-3,3′,7-trimethoxyflavon;

4′-Amino-5,7-dihydroxy-3-methoxyflavon;

4′-Amino-5-hydroxy-3,7-dimethoxyflavon;

3,3′,7-Trimethoxy-4′,5-bis-(nicotinoyloxy)-flavon;

4′,5-Bis-(ethoxycarbonyloxy)-3,3′,7-trimethoxyflavon;

5-Hydroxy-3,3′,7-trimethoxy-4′-(pivaloyloxy)-flavon;

5-(Isobutyryloxy)-3,3′,7-trimethoxy-4′-(pivaloyloxy)-flavon;

3,3′,7-Trimethoxy-4′,5-bis-(propionyloxy)-flavon;

3-Ethoxy-4′,5-dihydroxy-3′,7-dimethoxy-flavon;

4′-5-Dihydroxy-3-isopropoxy-3′-7-dimethoxy-flavon;

B. Bekannte Verbindungen

4′,5-Dihydroxy-3,3′,7-trimethoxyflavon
A.G. Valesi et al., Phytochemistry 11, 2821–6 (1972)

4′-Hydroxy-3,3′,5,7-tetramethoxyflavon
S. Yamaguchi, Nippon Kagaku Zasshi 81, 1332–6 (1960)

5,7-Dihydroxy-3,4′-dimethoxyflavon
S.K. Grover et al., Indian J. Chem. 1, 382–5 (1963)

3′,5-Dihydroxy-3,4′,7-trimethoxyflavon
L. Jurd, J. Org. Chem. 27, 1294–7 (1962)

4′,5-Dihydroxy-3,7-dimethoxyflavon
C.P. Bahl et al., Current Sci, (India) 35, 281 (1966)

5-Hydroxy-3,4′,7-trimethoxyflavon
H. Erdtman et al., Tetrahedron suppl. No. 8 pt. 1, 71–4 (1966)

4′,5-Dihydroxy-2′,3,7-trimethoxyflavon
4′,5-Diacetoxy-3,3′,7-trimethoxyflavon
K. Picker et al., Aust. J. Chem. 26, 1111–19 (1973)

Nach dem erfindungsgemässen Verfahren können die neuen 3-Alkoxyflavonderivate der Formel II dadurch hergestellt werden, dass man

(a) die Hydroxygruppe in 4'-Stellung oder die Hydroxygruppen in 1- und 4'-Stellung einer Verbindung der allgemeinen Formel

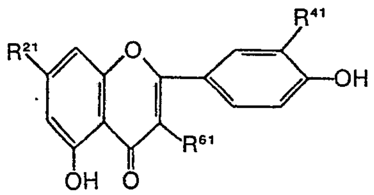

III

worin

$R^{21}$ $C_{1-4}$-Alkoxy; $R^{41}$ Wasserstoff oder $C_{1-4}$-Alkoxy und $R^{61}$ $C_{1-4}$-Alkoxy darstellen,

mit einem für eine Acylierung geeigneten reaktionsfähigen Derivat einer $C_{2-7}$-Alkancarbonsäure, von L-Lysin, L-Alanin, L-Glutamin, α-Glutamat, 1-Hydroxy-1,2-ethandicarbonsäure, der 1,2-Dihydroxy-1,2-ethandicarbonsäure oder der Nicotinsäure umsetzt oder

(b) die Hydroxygruppe in 4'-Stellung einer Verbindung der Formel III mit einem für eine Glycosilierung geeigneten reaktionsfähigen Derivat eines entsprechenden Monosaccharids glycosiliert oder

(c) ein 2',4',6'-Trihydroxy-2-alkoxyacetophenon mit einem Bis-(4-acetamidobenzoesäure)-anhydrid und einem Alkalimetallsalz der 4-Acetamidobenzoesäure behandelt und die erhaltene Verbindung mit einem Alkalimetallhydroxid zu einem 4'-Amino-5,7-dihydroxy-3-alkoxyflavon umsetzt und gewünschtenfalls durch weitere Umsetzung mit einem Diazoalkan in die entsprechende 7-Alkoxyverbindung überführt.

Die Acylierung gemäss Verfahrensvariante (a), die Glycosylierung gemäss Verfahrensvariante (b), sowie die Umsetzung gemäss Verfahrensvariante (c) des erfindungsgemässen Verfahrens können in an sich bekannter Weise durchgeführt werden und werden durch die später folgenden Beispiele illustriert.

Die beiden beanspruchten Gruppen von 3-Alkoxyflavonderivaten der Formeln I und II zeigen antivirale Aktivitäten, insbesondere hemmen sie die Replikation von menschlichen Rhino-Viren und Entero-Viren, wie ECHO-Viren, Coxsackie-Viren, Polio-Viren und dergleichen in menschlichen embryonalen Lungenzellen oder HeLa-Zellkulturen in Dosen von 0,05–10 µg/ml.

Das Studium der antiviralen Aktivität ergab folgende Resultate:

1. In vitro antivirale Aktivität

1) Hemmung des viralen cytopathogenen Effektes

Eine Suspension von HeLa-Zellen ($6 \times 10^4$) wird vermischt mit Rhino-Viren HGP ($3 \times 10^3$ koloniebildende Einheiten, PFU) oder Coxsackie-Viren B1 ($3 \times 10^3$ PFU) und auf einer Mikrotestplatte aufgetragen, die die zu testenden Verbindungen in einer Verdünnungsreihe enthält. Die Zellen werden anschliessend mit Eagle's essentiellem Minimalmedium kultiviert, das 2% Kälberserum, 1% Tryptosephosphatbrühe, 100 µg/ml Streptomycin und 20 Einheiten/ml Penicillin G enthält. Der virale cytopathogene Effekt wird unter dem Mikroskop beobachtet, und zwar nach einem Tag Kultur bei 37 °C für Coxsackie-Virusinfektion, und nach zwei Tagen Kultur bei 33 °C für Rhino-Virusinfektion.

Die erhaltenen Resultate sind in der Tabelle 1 zusammengefasst. Die antivirale Aktivität der getesteten Verbindungen wird angegeben als Konzentration, die nötig ist, um den viralen cytopathogenen Effekt, verglichen mit einer Kontrollkultur, um 50% zu hemmen ($IC_{50}$).

Tabelle 1

| Verbindung | $IC_{50}$ (µg/ml) | |
|---|---|---|
| | Rhino-Virus HGP | Coxsackie-Virus BI |
| 4',5-Dihydroxy-3,3',7-trimethoxyflavon | 0,1 | 0,1–0,2 |
| 4'Hydroxy-3,3',5,7-tetramethoxyflavon | 2–7 | 6–7 |
| 5,7-Dihydroxy-3,4'-dimethoxyflavon | 0,5 | >4 |
| 3',5-Dihydroxy-3,4',7-trimethoxyflavon | 3 | 3–10 |
| 4',5-Dihydroxy-3,7-dimethoxyflavon | 0,1 | 0,1 |
| 5-Hydroxy-3,4',7-trimethoxyflavon | 1 | >8 |
| 4',5-Dihydroxy-2',3,7-trimethoxyflavon | 0,3 | 1–3 |
| 4'-Acetoxy-5-hydroxy-3,3',7- -trimethoxyflavon | 0,5 | 0,25–0,5 |
| 5-Hydroxy-4'-(L-lysyloxy-3,3',7- -trimethoxyflavon | 0,25 | 0,25 |
| 4'-(β-D-Glucopyranosyloxy)-5-hydroxy-3,3',7- -trimethoxyflavon | 0,25 | 0,25–0,5 |
| 4'-(L-Alanyloxy)-5-hydroxy-3,3',7- -trimethoxyflavon | 0,5 | 0,25–0,5 |
| 4'-(L-Glutaminyloxy)-5-hydroxy-3,3'7- -trimethoxyflavon | 0,25–0,5 | 0,25–0,5 |
| 4'-L-α-Glutamyloxy)-5-hydroxy-3,3',7- -trimethoxyflavon | 0,25 | 0,25–0,5 |
| 4'-(3-Carboxy-2,3-dihydroxypropionyloxy)- -5-hydroxy-3,3',7-trimethoxyflavon | 0,1–0,2 | 0,1–0,2 |
| 4'-Amino-5,7-dihydroxy-3-methoxyflavon | 1–3 | 3–10 |

Tabelle 1 (Fortsetzung)

| Verbindung | $IC_{50}$ (µg/ml) | |
|---|---|---|
| | Rhino-Virus HGP | Coxsackie-Virus BI |
| 4′-Amino-5-hydroxy-3,7-dimethoxyflavon | 3 | 10–30 |
| 3,3′,7-Trimethoxy-4′,5-bis-(nicotinoyloxy)--flavon | 0,3–1 | 0,3–1 |
| 4′,5-Diacetoxy-3,3′,7-trimethoxyflavon | 0,1–0,2 | 0,1–0,2 |
| 4′,5-Bis-(ethoxycarbonyloxy)-3,3′,7--trimethoxyflavon | 0,3–1 | 0,3–1 |
| 5-Hydroxy-3,3′,7-trimethoxy-4′--(pivaloyloxy)-flavon | 0,3 | 0,3–1 |
| 5-(Isobutyryloxy)-3,3′,7-trimethoxy-4′--(pivaloyloxy)-flavon | 0,3 | 0,3–1 |
| 3,3′,7-Trimethoxy-4′,5-bis-(propionyloxy)-flavon | 0,1–0,2 | 0,1–1,2 |
| 3-Ethoxy-4′,5-dihydroxy-3′,7-dimethoxy-flavon | 0,05–0,1 | 0,05–0,1 |
| 4′,5-Dihydroxy-3-isopropoxy-3′,7-dimethoxy flavon | 0,05–0,1 | 0,05–0,1 |

Ausserdem zeigt die Tabelle 2 die in vitro Wirkungsspektren von 4′,5-Dihydroxy-3,3′,7-trimethoxyflavon (A) und 4′,5-Dihydroxy-3,7-dimethoxyflavon (B).

Tabelle 2

| Virus Stamm | Typ | minimale Hemmkonzentration (A) | (µg/ml (B) |
|---|---|---|---|
| Rhino-Virus | 1A | 0,03 | 0,03 |
| Rhino-Virus | 2 | 0,1 | 0,1 |
| Rhino-Virus | 3 | 0,1 | 0,3 |
| Rhino-Virus | 4 | 1,0 | 0,3–1,0 |
| Rhino-Virus | 9 | 0,1 | 0,3 |
| Rhino-Virus | 13 | 0,3–1,0 | 0,3–1,0 |
| Rhino-Virus | 14 | 0,1 | 0,3 |
| Rhino-Virus | 16 | 0,3 | 0,3–1,0 |
| Rhino-Virus | 17 | 0,3 | 0,1 |
| Rhino-Virus | 21 | 0,3 | 0,3 |
| Rhino-Virus | 26 | 0,3 | 0,3 |
| Rhino-Virus | 30 | 0,03–0,1 | 0,1 |
| Rhino-Virus | 32 | 0,1 | 0,3 |
| Rhino-Virus | 34 | 0,3 | 0,3 |
| Rhino-Virus | 36 | 0,3 | 0,3 |
| Rhino-Virus | 39 | 0,1 | 0,3 |
| Rhino-Virus | 44 | 0,05–0,5 | 0,5 |
| Rhino-Virus | 47 | 0,3 | 0,3 |
| Rhino-Virus | 50 | 0,3 | 0,3 |
| Rhino-Virus | 53 | 0,1 | 0,3 |
| Rhino-Virus | 55 | 0,3 | 0,1–0,3 |
| Coxsackie-Virus A | 21 | 0,1–0,3 | – |
| Coxsackie-Virus B | 1 | 0,1–0,2 | 0,1 |
| ECHO-Virus | 9 | 0,1–0,3 | – |
| ECHO-Virus | 11 | 0,1–0,3 | – |
| ECHO-Virus | 12 | 0,1–0,3 | – |
| ECHO-Virus | 19 | 0,1–0,3 | – |
| Polio-Virus | 1 | 0,1–0,3 | – |

2) Hemmung der viralen Replikation

Es wird der Effekt von 4′,5-Dihydroxy-3,3′,7-trimethoxyflavon auf die Replikation von Rhino-Viren HGP, 1A und Coxsackie-Viren B1 in HeLa Zellen getestet. Monoschichten der besagten Zellen ($4 \times 10^5$) werden mit jedem Virus ($4 \times 10^4$

PFU) während 60 Minuten infiziert. Anschliessend werden die Zellen mit Eagle's essentiellem Minimalmedium gewaschen und weiter im erwähnten Medium kultiviert, das 2% Kälberserum, 1% Tryptosephosphatbrühe, 100 µg/ml Streptomycinsulfat, 20 Einheiten/ml Penicillin G und 4',5-Dihydroxy-3,3',7-trimethoxyflavon in variablen Mengen enthält. Die Gesamtmenge der replizierten Rhino-Viren bzw. Coxsackie-Viren in den Kulturen werden zwei Tage bzw. einen Tag nach der Infektion bestimmt.

Die dabei erhaltenen Resultate sind in der Figur 1 dargestellt, und zeigen, dass 4',5-Dihydroxy-3,3',7-trimethoxyflavon, in Konzentrationen von 0,5–2 µg/ml, die virale Replikation beträchtlich reduziert. Bei diesen Konzentrationen wird das HeLa-Zellwachstum nicht beeinträchtigt.

2. In vivo antivirale Aktivität

1) Anti-Coxsackie-Virus-Aktivität

Die in der Tabelle 3 aufgeführten erfindungsgemässen Verbindungen werden auf ihre antivirale Aktivität gegen letale Infektionen mit Coxsackie-Virus B1 in Mäusen geprüft. Die 15 g schweren ddy-Mäuse werden intraperitoneal mit etwa der 10-fachen $LD_{50}$ von Coxsackie-Viren B1 infiziert. Die infizierten Mäuse werden anschliessend vier- oder neunmal, d.h. 2, 6, 18 und 30 Stunden oder 0, 2, 5, 18, 24, 42, 48, 66 und 72 Stunden nach der Infektion, intraperitoneal, intravenös oder oral mit den Testsubstanzen behandelt. Die überlebenden Mäuse werden nach 21 Tagen gezählt.

Die so erhaltenen Resultate sind in der Tabelle 3 zusammengefasst. Kontrollmäuse, welche mit Phosphat-gepufferter Kochsalzlösung oder Wasser behandelt werden, sterben 3 bis 5 Tage nach der Infektion.

Tabelle 3

| Verbindung | Dosis | Verabreichung | Überlebende (%) |
|---|---|---|---|
| 4',5-Dihydroxy-3,3',7-trimethoxyflavon | 20 mg/kg × 9[1] | i.p. | 20 |
| | 10 mg/kg × 9 | i.p. | 10 |
| | 40 mg/kg × 9 | p.o. | 30 |
| | 20 mg/kg × 9 | p.o. | 10 |
| 5-Hydroxy-4'-(L-lysyloxy)-3,3',7-trimethoxyflavon | 10 mg/kg × 9 | i.v. | 54 |
| 4'-(L-Alanyloxy)-5-hydroxy-3,3',7-trimethoxyflavon | 10 mg/kg × 9 | i.v. | 42 |
| 4'-(L-Glutaminyloxy)-5-hydroxy-3,3',7-trimethoxyflavon | 10mg/kg × 9 | i.v. | 54 |
| 3,3',7-Trimethoxy-4',5-bis (nicotinoyloxy)flavon | 20 mg/kg × 9 | p.o. | 30 |
| | 10 mg/kg × 9 | p.o. | 40 |
| 4',5-Diacetoxy-3,3',7-trimethoxyflavon | 20 mg/kg × 9 | i.p. | 67 |
| | 10 mg/kg × 9 | i.p. | 54 |
| | 80 mg/kg × 4[2] | p.o. | 60 |
| | 40 mg/kg × 4 | p.o | 50 |
| | 40 mg/kg × 9 | p.o. | 50 |
| | 20 mg/kg × 9 | p.o. | 20 |
| 4',5-Bis(ethoxycarbonyloxy)- | 20 mg/kg × 9 | p.o. | 30 |
| 3,3',7-trimethoxyflavon | 10 mg/kg × 9 | p.o. | 30 |
| 5-(Isobutyryloxy)-3,3',7- | 20 mg/kg × 9 | p.o. | 40 |
| trimethoxy-4'-(pivaloyloxy)flavon | 10 mg/kg × 9 | p.o. | 30 |
| keine | – | – | 0 |

Die folgende Tabelle 4 zeigt die antivirale Aktivität von 4',5-Diacetoxy-3,3',7-trimethoxyflavon gegen Infektionen mit verschiedenen Mengen an Coxsackie-Viren B1 in Mäusen. Die Verbindung, in einer Lösung von 0,5% Carboxymethylcellulose suspendiert, wird oral 0, 2, 5, 18, 24, 42, 48, 66 und 72 Stunden nach der letalen Infektion mit Coxsacki-Viren B1 (i.p.) verabreicht. Die überlebenden Mäuse werden nach 21 Tagen gezählt.

Tabelle 4

| Verabreichung mit obengenannter Verbindung | Überlebende/Total | | | |
|---|---|---|---|---|
| Infektion mit Coxsackie-Virus BI ($Ld_{50}$) | 2,5 × | 10 × | 40 × | 160 × |
| 40 mg/kg × 9 | 4/7 | 3/7 | 2/7 | 2/7 |
| 20 mg/kg × 9 | 3/7 | 2/7 | 1/7 | 1/7 |
| 10 mg/kg × 9 | 2/7 | 1/7 | 0/7 | 0/7 |
| 0 | 0/7 | 0/7 | 0/7 | 0/7 |

2) Anti-Influenza-Virus-Aktivität

4',5-Diacetoxy-3,3',7-trimethoxyflavon wird auf eine Aktivität gegen Influenza-Virus A2/Adachi in Mäusen getestet. Die 12 g schweren ddy-Mäuse werden intranasal mit etwa der 5-fachen $LD_{50}$ von Influenza-Virus infiziert. Die Mäuse werden neunmal mit der Verbindung intraperitoneal behandelt, und die Überlebenden werden nach 21 Tagen gezählt.

Tabelle 5

| Behandlung | Überlebende (%) | |
|---|---|---|
| Phosphat-gepufferte Kochsalzlösung | | |
| 4',5-Diacetoxy-3,3',7-trimethoxyflavon | 0/8 | ( 0) |
| 20 mg/kg × 9,* i.p. | 2/7 | (29) |
| 40 mg/kg × 9, i.p. | 3/8 | (38) |
| Amantadin | | |
| 20 mg/kg × 9 i.p. | 0/6 | ( 0) |
| 40 mg/kg × 9, i.p. | 2/7 | (29) |

* verabreicht 0,5 Stunden vor und 4, 8, 12, 24, 28, 32, 48 und 56 Stunden nach der Infektion

Wie aus der Tabelle 5 hervorgeht, ist die antivirale Aktivität der Verbindung mindestens so hoch wie diejenige von Amantadin, einem konventionellen Anti-Influenza-Virus-Mittel.

Darüber hinaus werden die erfindungsgemässen Verbindungen gut vertragen und zeigen keinerlei Cytotoxizität bei Konzentrationen, die 10–100 × höher sind als die Konzentrationen, die eine antivirale Aktivität bewirken. Bei oraler Verabreichung an Mäusen, bewirken die Verbindungen keine toxischen Symptome bei Dosen von 5 g/kg. Die folgende Tabelle 6 enthält Angaben über die akute Toxizität für Mäuse.

Tabelle 6

| Verbindung | $LD_{50}$ (mg/kg)[1] i.p.[2] | p.o.[2] |
|---|---|---|
| 4',5-Dihydroxy-3,3', 7-trimethoxyflavon | >1,000 | >5,000 |
| 5-Hydroxy-4'-(L-lysyloxy)-3,3',7-trimethoxyflavon | 1,000 | >5,000 |
| 4'-(L-Alanyloxy)-5-hydroxy-3,3',7-trimethoxyflavon | >1,000 | >5,000 |
| 4'-(L-Glutaminyloxy)-5-hydroxy-3,3', 7-trimethoxyflavon | >1,000 | >5,000 |
| 4',5-Diacetoxy-3,3', 7-trimethoxyflavon | >1,000 | >5,000 |

[1] Den 15–20 g schweren ddy-Mäusen wird eine einmalige Dosis der entsprechenden Verbindung verabreicht. Die Überlebenden werden nach 21 Tagen gezählt.

[2] Die Verbindungen werden in Dimethylsulfoxid gelöst.

[3] Die Verbindungen werden in einer Lösung von 0,5% Carboxymethylcellulose suspendiert und mit Ultraschall behandelt.

Wie bereits oben erwähnt können Verbindungen der Formeln I und II als Medikamente gegen virale Erkrankungen verwendet werden, die durch Rhino-Viren, Entero-Viren, Influenza-Viren und dergleichen verursacht werden; und zwar als Bestandteil von pharmazeutischen Präparaten.

Die pharmazeutischen Präparate enthalten mindestens eine der erwähnten antiviral wirksamen Verbindungen zusammen mit einem verträglichen pharmazeutischen Träger. Dieser Träger kann ein für die enterale, perkutane oder parenterale Verabreichung geeignetes organisches oder anorganisches Trägermaterial sein, wie z.B. Wasser, Gelatine, Gummi arabicum, Lactose, Stärke, Magnesiumstearat, Talk, pflanzliche Öle, Polyalkylenglykole, Vaseline und dergleichen. Darüber hinaus können die pharmazeutischen Präparate weitere pharmazeutisch wertvolle Stoffe enthalten, wie fiebersenkende Mittel, schmerzstillende Mittel, entzündungshemmende Mittel, Antihistamine, Interferonstarter und dergleichen. Die pharmazeutischen Präparate können oral, z.B. in Form von Tabletten, Kapseln, Pillen, Pulver, Granulaten, Lösungen, Sirupen, Suspensionen, Elixiren und dergleichen verabreicht werden. Die Verabreichung kann aber auch parenteral, z.B. in Form von sterilen Lösungen, Suspensionen oder Emulsionen, oder lokal, in Form von Lösungen, Suspensionen, Salben, Pudern, Aerosolen und dergleichen, erfolgen. Die pharmazeutischen Präparate können sterilisiert sein und/oder Bestandteile enthalten, wie Konservierungsmittel, Stabilisatoren, Netzmittel, Emulgatoren, Salze, um den osmotischen Druck zu variieren, und Puffersubstanzen.

Die pharmazeutischen Präparate können so verabreicht werden, dass die Konzentration des aktiven Wirkstoffes grösser ist als die erforderliche minimale Hemmkonzentration.

Die Behandlungsdosis ist abhängig von der Verabreichungsart, vom Alter, Gewicht und Befinden des Patienten und insbesondere von der Krankheit, die behandelt werden soll. Typische Dosierungen für Erwachsene sind z.B. 100–1000 mg, drei- bis sechsmal täglich oral oder parenteral verabreicht und 0,1–100 µg/cm², drei- bis sechsmal täglich lokal verabreicht.

Die folgenden Beispiele sollen die vorliegende Erfindung erläutern:

Beispiel 1

Eine Mischung aus 0,67 g 2',6'-Dihydroxy-2,4'-dimethoxyacetophenon, 3,5 g Bis-[4-(benzyloxy)-3-methoxybenzoesäure]-anhydrid und 1 g Natrium 4-(Benzyloxy)-3-methoxybenzoat wird während 3 Stunden unter vermindertem Druck auf 180–185 °C erwärmt. Nach Abkühlen gibt man 12 ml einer 10 prozentigen alkoholischen Kaliumhydroxidlösung hinzu, und erhitzt die Mischung unter Stickstoff während 30 Minuten zum Rückfluss. Die abgekühlte Mischung wird mit 20 ml 1N Salzsäure versetzt und mit 100 ml Chloroform ausgeschüttelt. Die organische Phase wird abgetrennt und im Vakuum eingedampft. Der Rückstand wird an Silicagel mit Chloroform chromatographiert. Nach Umkristallisieren aus Essigester/Hexan erhält man 0,86 g (60%) 4'-(Benzyloxy)-5-hydroxy-3,3',7-trimethoxyflavon als gelbe Kristalle vom Schmelzpunkt 156–157 °C.

Eine Lösung von 0,86 g 4'-(Benzyloxy)-5-hydroxy-3,3',7-trimethoxyflavon in 30 ml Äthanol wird mit 50 mg 5% Palladium auf Kohle, bei Raumtemperatur und Normaldruck hydriert. Nach 1 Stunde wird vom Katalysator abfiltriert und im Vakuum eingedampft. Der Rückstand wird aus Essigester/Hexan umkristallisiert. Man erhält 0,58 g (90%) 4',5-Dihydroxy-3,3',7-trimethoxyflavon als gelbe Kristalle vom Schmelzpunkt 171–173 °C.

Beispiel 2

Aus 2'-Hydroxy-2,4',6'-trimethoxyacetophenon erhält man in Analogie zu Beispiel 1 4'-Hydroxy-3,3',5,7-tetramethoxyflavon vom Schmelzpunkt 221–223 °C (Ausbeute 24%).

Beispiel 3

Aus 2',4',6-Trihydroxy-2-methoxyacetophenon, Bis-(4-methoxybenzoesäure)-anhydrid und Natrium-4-methoxybenzoat erhält man in Analogie zu Beispiel 1 5,7-Dihydroxy-3,4'-dimethoxyflavon vom Schmelzpunkt 238–239 °C (Ausbeute 40%).

Beispiel 4

In Analogie zu Beispiel 1 erhält man aus Bis-[3-(benzyloxy)-4-methoxybenzoesäure]-anhydrid und Natrium 3-(benzyloxy)-4-methoxybenzoat 33% 3',5-Dihydroxy-3,4',7-trimethoxyflavon vom Schmelzpunkt 171 °C.

Beispiel 5

In Analogie zu Beispiel 1 erhält man aus Bis-(4-benzyloxy-benzoesäure)-anhydrid und Natrium-4-benzyloxybenzoat 33% 4',5-Dihydroxy-3,7-dimethoxyflavon vom Schmelzpunkt 252–253 °C.

Beispiel 6

In Analogie zu Beispiel 1 erhält man aus Bis-(4-methoxybenzoesäure)-anhydrid und Kalium-4-methoxybenzoat 37% 5-Hydroxy-3,4',7-trimethoxyflavon vom Schmelzpunkt 143–145 °C.

Beispiel 7

In Analogie zu Beispiel 1 erhält man aus Bis-[4-(benzyloxy)-2-methoxybenzoesäure]-anhydrid und Natrium-4-(benzyloxy)-2-methoxybenzoat 50% 4',5-Dihydroxy-2',3,7-trimethoxyflavon vom Schmelzpunkt 191–192 °C.

Beispiel 8

Eine Mischung aus 250 mg 4',5-Dihydroxy-3,3',7-trimethoxyflavon, 60 mg Natriumacetat und 70 mg Essigsäureanhydrid wird während 2 Stunden auf 100 °C erwärmt. Nach Eindampfen der Reaktionsmischung wird der Rückstand mit 30 ml Chloroform extrahiert. Das Lösungsmittel wird entfernt, und der Rückstand mehrmals aus Methanol umkristallisiert. Dabei erhält man 250 mg (90%) 4'-Acetoxy-5-hydroxy-3,3',7-trimethoxyflavon als gelbe Kristalle vom Schmelzpunkt 168–169 °C.

Beispiel 9

Eine Lösung von 460 mg 4',5-Dihydroxy-3,3',7-trimethoxyflavon und 600 mg N,N'-Di-(benzyloxycarbonyl)-L-lysin in 5 ml Pyridin wird auf −10 bis −5 °C abgekühlt. Über einen Zeitraum von 5 Minuten gibt man unter Rühren 0,21 ml Thionylchlorid hinzu und lässt während 3 Tagen bei −5 °C stehen. Anschliessend gibt man 30 ml Wasser hinzu, und extrahiert die Mischung mit 50 ml Chloroform. Die organische Phase wird über Natriumsulfat getrocknet und im Vakuum eingedampft. Der Rückstand wird an Silicagel chromatographiert, unter Eluieren mit Chloroform. Dabei erhält man 920 mg des N,N'-Di-(benzyloxycarbonyl)-L-lysylesters von 4',5-Dihydroxy-3,3',7-trimethoxyflavon.

920 mg dieses Esters werden in 3 ml Essigsäure gelöst, die 25% Bromwasserstoff enthält. Nach 45 Minuten bei Raumtemperatur wird die Mischung lyophilisiert und ergibt 780 mg 5-Hydroxy-4'-(L-lysyloxy)-3,3',7-trimethoxyflavon als schwach gelbes Pulver vom Schmelzpunkt 164 °C (Zers.).

Beispiel 10

Eine eisgekühlte Lösung von 250 mg 4',5-Dihydroxy-3,3',7-trimethoxyflavon in 10 ml Aceton wird abwechslungsweise mit einer Lösung von 400 mg 2,3,4,6-tetra-O-Acetyl-α-D-glucopyranosylbromid in 10 ml Aceton und 5 ml 0,8 prozentiger wässriger Natronlauge unter Rühren über einen Zeitraum von 30 Minuten versetzt. Nach 3 Stunden bei Raumtemperatur gibt man 20 ml 0,2 prozentiger Natronlauge hinzu und rührt während weiteren 3 Stunden bei Raumtemperatur. Die entstandenen Kristalle werden abfiltriert und aus Ethanol umkristallisiert. Dabei erhält man 390 mg (80%) 4'-(β-D-Glucopyranosyloxy)-5-hydroxy-3,3',7-trimethoxyflavon als schwach gelbe Nadeln vom Schmelzpunkt 203–204 °C.

Beispiel 11

Eine Lösung von 500 mg 4',5-Dihydroxy-3,3',7-trimethoxyflavon und 360 mg N-(Benzyloxycarbonyl)-L-alanin in 5 ml Pyridin wird auf −10 bis −5 °C gekühlt. Über einen Zeitraum von 15 Minuten gibt man unter Rühren 0,38 g Thionylchlorid hinzu, und lässt die Reaktionsmischung während 3 Stunden bei −5 °C stehen. Nach Zugabe von 30 ml Wasser wird die Mischung mit 50 ml Chloroform extrahiert. Die organische Phase wird über Natriumsulfat getrocknet und im Vakuum eingedampft. Der Rückstand wird an Silicagel unter Eluieren mit Chloroform gereinigt. Dabei erhält man 640 mg des N-(Benzyloxycarbonyl)-L-alanylesters von 4′,5-Dihydroxy-3,3′,7-trimethoxyflavon.

640 mg dieses Esters werden in 3 ml Essigsäure gelöst, die 25% Bromwasserstoff enthält. Die Lösung wird während 45 Minuten bei Raumtemperatur stehengelassen und anschliessend lyophilisiert. Das so erhaltene gelbe Pulver wird dreimal mit je 10 ml Methylenchlorid gewaschen. Das unlösliche Material wird im Vakuum über Phosphorpentoxid getrocknet und ergibt 370 mg (64%) 4′-(L-Alanyloxy)-5-hydroxy-3,3′,7-trimethoxyflavon-hydrobromid vom Schmelzpunkt 201–203 °C.

Beispiel 12

Eine Lösung von 500 mg 4′,5-Dihydroxy-3,3′,7-trimethoxyflavon und 450 mg N-(Benzyloxycarbonyl)-L-glutamin in 5 ml Pyridin wird auf −10 bis −5 °C abgekühlt. Über einen Zeitraum von 15 Minuten gibt man 200 mg Thionylchlorid unter Rühren hinzu und lässt die Reaktionsmischung während 3 Stunden bei −5 °C stehen. Man gibt 30 ml Wasser hinzu und schüttelt die erhaltene Mischung mit 50 ml Chloroform aus. Der Extrakt wird über Natriumsulfat getrocknet und im Vakuum eingedampft. Der Rückstand wird an Silicagel chromatographiert, unter Eluieren mit Chloroform/Methanol (9:1, v/v) und ergibt 830 mg des N-(Benzyloxycarbonyl)-L-glutaminylesters von 4′,5-Dihydroxy-3,3′,7-trimethoxyflavon.

830 mg dieses Esters werden in 3 ml Essigsäure gelöst, die 25% Bromwasserstoff enthält. Man lässt die Lösung während 45 Minuten bei Raumtemperatur stehen, lyophilisiert, und wäscht den Rückstand mit Methylenchlorid. Dabei erhält man 750 mg (98%) 4′-(L-Glutaminyloxy)-5-hydroxy-3,3′,7-trimethoxyflavon-hydrobromid vom Schmelzpunkt 185–188 °C.

Beispiel 13

Eine Lösung von 500 mg 4′,5-Dihydroxy-3,3′,7-trimethoxyflavon und 600 mg 5-p-Nitrobenzyl-N-(benzyloxycarbonyl)-L-glutamat in 5 ml Pyridin wird auf −10 bis −5 °C abgekühlt. Über einen Zeitraum von 15 Minuten gibt man 0,17 g Thionylchlorid unter Rühren hinzu und lässt die Reaktionsmischung während 3 Stunden bei −5 °C stehen. Anschliessend versetzt man mit 30 ml Wasser, schüttelt die Mischung mit 50 ml Chloroform aus, trocknet den Extrakt über Natriumsulfat und dampft im Vakuum ein. Der Rückstand wird an Silicagel chromatographiert, mit Essigester/Hexan (1:1, v/v) als Elutionsmittel. Man erhält so 670 mg schwach gelbe Kristalle.

670 mg dieses Materials, gelöst in 50 ml Chloroform, werden an 50 mg Palladiumschwarz bei Raumtemperatur und unter Normaldruck während 3 Stunden hydriert. Anschliessend wird vom Katalysator abfiltriert und eingedampft. Der Rückstand wird nach Waschen mit 10 ml Methylenchlorid in 3 ml Essigsäure gelöst, die 25% Bromwasserstoff enthalten. Nach 45 Minuten bei Raumtemperatur wird lyophilisiert und der Rückstand dreimal mit je 10 ml Methylenchlorid gewaschen. Dabei erhält man 370 mg (84%) 4′-(L-α-Glutamyloxy)-5-hydroxy-3,3′,7-trimethoxyflavon-hydrobromid vom Schmelzpunkt 243–246 °C.

Beispiel 14

Eine Lösung aus 500 mg 4′,5-Dihydroxy-3,3′,7-trimethoxyflavon, 470 mg Mono-p-methoxybenzylester von 2,3-O-Isopropylidenweinsäure und einem Tropfen Dimethylformamid in 5 ml Pyridin wird auf −10 bis −5 °C gekühlt. Dazu gibt man über einen Zeitraum von 15 Minuten 170 mg Thionylchlorid, und lässt die Mischung bei −5 °C während 3 Stunden stehen. Anschliessend gibt man 30 ml Wasser hinzu, schüttelt die Mischung mit 50 ml Chloroform aus, trocknet den Extrakt über Natriumsulfat, filtriert und dampft ein. Der Rückstand wird an Silicagel chromatographiert, unter Eluieren mit Chloroform, wobei man 520 mg eines schwach gelben Festkörpers erhält.

Dieser Festkörper wird, gelöst in 50 ml Chloroform, an 50 mg Palladiumschwarz bei Raumtemperatur und Normaldruck während 3 Stunden hydriert. Anschliessend wird vom Katalysator abfiltriert und zur Trockne eingedampft. Der Rückstand wird nach Waschen mit Methylenchlorid in 3 ml Essigsäure gelöst, die 25% Bromwasserstoff enthält. Nach 45 Minuten bei Raumtemperatur wird lyophilisiert und der Rückstand dreimal mit je 10 ml Methylenchlorid gewaschen. Man erhält 280 mg (74%) 4′-(3-Carboxy-2,3-dihydroxypropionyloxy)-5-hydroxy-3,3′,7-trimethoxyflavon vom Schmelzpunkt 170–180 °C.

Beispiel 15

Eine Mischung aus 847 mg 2′,4′,6′-trihydroxy-2-methoxyacetophenon, 5,82 g Bis-(4-acetamidobenzoesäure)-anhydrid und 1,2 g Natrium 4-acetamidobenzoat wird unter vermindertem Druck während 3 Stunden auf 230 °C erhitzt. Nach Abkühlen gibt man 90 ml Methanol und 40 ml 40 prozentige wässrige Kalilauge hinzu und erhitzt die Mischung während 1 Stunde zum Rückfluss. Nach Entfernen des Methanols im Vakuum wird der wässrige Rückstand mit 200 ml Wasser verdünnt und die erhaltene Suspension filtriert. Das Filtrat wird mit Kohlendioxid gesättigt und dreimal mit je 150 ml Essigester ausgeschüttelt. Die vereinigten organischen Phasen werden mit Wasser gewaschen, über Natriumsulfat getrocknet und im Vakuum eingedampft. Man erhält 460 mg Rohprodukt.

Der Rückstand wird an 15 g Silicagel chromatographiert unter Eluieren mit Essigester/Hexan (1:1, v/v). Durch Eindampfen von 120 ml Eluat im Vakuum erhält man einen gelben Rückstand. Nach Umkristallisieren aus Methanol resultieren 17 mg 4′-Amino-5,7-dihydroxy-3-methoxyflavon als schwach gelbe Kristalle: $^1$H-NMR-Spektrum (in DMSO-$d^6$), 3,75(3H), 5,98(2H), 6,16(1H), 6,41(1H),

6,68(2H), 7,83(2H), 11(1H, breit) und 12,82 ppm(H).

Die Säule wird anschliessend mit Aceton/Methanol (1:1, v/v) eluiert. Nach Entfernen des Lösungsmittels wird der Rückstand mit einer ätherischen Diazomethanlösung behandelt. Nach Stehen über Nacht bei Raumtemperatur wird die Lösung eingedampft und ergibt 400 mg eines Festkörpers.

Der Rückstand wird an 18 g Silicagel unter Eluieren mit Essigester/Hexan chromatographiert, wobei man Fraktionen zu 30 ml sammelt. Die Fraktionen Nr. 3–9 werden vereinigt, im Vakuum eingedampft und ergeben einen gelben kristallinen Rückstand. Durch Umkristallisieren aus Methanol erhält man 129 mg 4′-Amino-5-hydroxy-3,7-dimethoxyflavon als gelbe Kristalle vom Schmelzpunkt 221 °C.

Beispiel 16

Eine in einem Eisbad gekühlte Lösung von 200 mg (0,58 mMol) 4′,5-Dihydroxy-3,3′,7-trimethoxyflavon in 10 ml Pyridin wird über einen Zeitraum von 10 Minuten unter Rühren mit 220 mg (1,2 mMol) Nicotinoylchlorid-hydrochlorid versetzt. Nach 3-stündigem Rühren bei Raumtemperatur wird die Mischung im Vakuum eingedampft, wobei ein öliger Rückstand anfällt.

Der Rückstand wird in 30 ml Chloroform aufgenommen und nacheinander zweimal mit je 20 ml gesättigter Natriumbicarbonatlösung und 20 ml Wasser gewaschen. Die Lösung wird über Natriumsulfat getrocknet und eingedampft, dabei erhält man 200 mg eines Festkörpers. Durch Umkristallisieren aus Benzol erhält man 170 mg (53%) 3,3′,7-Trimethoxy-4′,5-bis-(nicotinoyloxy)-flavon als farblose Nadeln vom Schmelzpunkt 212–214 °C.

Beispiel 17

In Analogie zu Beispiel 16 erhält man mit Propionylchlorid 47% 3,3′,7-Trimethoxy-4′,5-bis-(propionyloxy)-flavon vom Schmelzpunkt 106–107 °C.

Beispiel 18

In Analogie zu Beispiel 16 erhält man mit Acetylchlorid rohes 4′,5-Diacetoxy-3,3′,7-trimethoxyflavon, das nach Umkristallisieren aus Essigester/Hexan reines Produkt vom Schmelzpunkt 165–166 °C liefert (Ausbeute 90%).

Beispiel 19

In Analogie zu Beispiel 16 erhält man mit Ethylchlorformiat rohes 4′,5-Bis-(ethoxycarbonyloxy)-3,3′,7-trimethoxyflavon, das nach Umkristallisieren aus Essigester/Hexan bei 106–107 °C schmilzt (Ausbeute 95%).

Beispiel 20

Eine Lösung von 0,2 g 4′,5-Dihydroxy-3,3′,7-trimethoxyflavon in 3 ml Pyridin wird unter Rühren mit 0,15 ml Pivaloylchlorid versetzt. Die Mischung wird während 3 Stunden bei Raumtemperatur gerührt und anschliessend noch während 1 Stunde auf 75 °C erwärmt. Nach dem Abkühlen dampft man ein und erhält einen öligen Rückstand.

Der Rückstand wird in 10 ml Ethanol/Hexan (1:1, v/v) gelöst und während der Nacht in einem Kühlschrank stehengelassen. Die so erhaltenen Kristalle werden abfiltriert, mit Hexan gewaschen und getrocknet. Man erhält 200 mg 5-Hydroxy-3,3′,7-trimethoxy-4′-(pivaloyloxy)-flavon als gelbe Nadeln vom Schmelzpunkt 163–164 °C.

Beispiel 21

Eine Lösung von 0,2 g 5-Hydroxy-3,3′,7-trimethoxy-4′-(pivaloyloxy)-flavon in 3 ml Pyridin wird unter Rühren mit 0,05 ml Isobutyrylchlorid versetzt und während 3 Stunden bei Raumtemperatur stehengelassen. Nach Entfernen des Lösungsmittels dampft man im Vakuum ein, nimmt den öligen Rückstand in 5 ml Ethanol/Hexan (1:1, v/v) auf, und lässt die Lösung über Nacht in einem Kühlschrank stehen. Die so erhaltenen Kristalle werden abfiltriert, mit Hexan gewaschen und getrocknet. Man erhält 200 mg 5-(Isobutyryloxy)-3,3′,7-trimethoxy-4′-(pivaloyloxy)-flavon als farblose Nadeln vom Schmelzpunkt 151–152 °C.

Beispiel 22

In Analogie zu Beispiel 1 erhält man aus 2-Ethoxy-2′,6′-dihydroxy-4′-methoxyacetophenon 3-Ethoxy-4′,5-dihydroxy-3′,7-dimethoxyflavon als schwach gelbes Pulver. Durch Umkristallisieren aus Ethanol erhält man reines Produkt als gelbe Nadeln vom Schmelzpunkt 168–169 °C.

Beispiel 23

In Analogie zu Beispiel 1 erhält man aus 2′,6′-Dihydroxy-2-isopropoxy-4′-methoxyacetophenon 4′,5-Dihydroxy-3-isopropoxy-3′,7-dimethoxyflavon als schwach gelbes Pulver. Durch Umkristallisieren aus Äthanol erhält man reines Produkt als gelbe Nadeln vom Schmelzpunkt 169–171 °C.

**Patentansprüche**

1. 3-Alkoxyflavonderivate der allgemeinen Formel

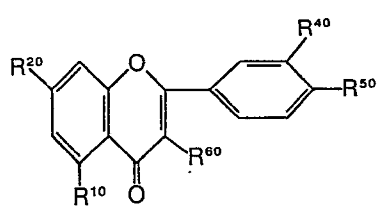

II

worin

$R^{10}$ Hydroxy, $C_{2-7}$-Alkanoyloxy, $C_{2-7}$-Alkoxycarbonyloxy oder Nicotinoyloxy;

$R^{20}$ Hydroxy oder $C_{1-4}$-Alkoxy; $R^{40}$ Wasserstoff oder $C_{1-4}$ Alkoxy; $R^{50}$ Hydroxy, $C_{2-7}$-Alkanoyloxy, L-Lysyloxy, L-Alanyloxy, L-Glutaminyloxy, α-Glutamyloxy, Glucosyloxy, Mannosyloxy, Galaktosyloxy, einen 1-Hydroxy-1,2-ethandicarbonsäurerest, einen 1,2-Dihydroxy-1,2-ethandicarbonsäurerest, Amino, Nicotinoyloxy oder $C_{2-7}$-Alkoxycarbonyloxy und

$R^{60}$ $C_{1-4}$-Alkoxy darstellen, mit der Einschränkung, dass $R^{50}$ nicht Acetoxy darstellt, wenn $R^{10}$ Acetoxy ist, und dass $R^{60}$ nicht Methoxy darstellt, wenn $R^{50}$ Hydroxy ist.

2. Eine der nachfolgenden Verbindungen gemäss Anspruch 1:
4'-Acetoxy-5-hydroxy-3,3',7-trimethoxyflavon,
4'-(3-Carboxy-2,3-dihydroxypropionyloxy)-5-hydroxy-3,3',7-trimethoxyflavon,
3,3',7-Trimethoxy-4',5-bis-(nicotinoyloxy)-flavon,
4',5-Bis-(ethoxycarbonyloxy)-3,3',7-trimethoxyflavon
5-Hydroxy-3,3',7-trimethoxy-4'-(pivaloyloxy)-flavon,
5-(Isobutyryloxy)-3,3',7-trimethoxy-4'-(pivaloyloxy) flavon,
3,3',7-Trimethoxy-4',5-bis-(propionyloxy)-flavon.

3. Eine der nachfolgenden Verbindungen gemäss Anspruch 1:
5-Hydroxy-4'-(L-lysyloxy)-3,3',7-trimethoxyflavon,
4'-(L-Alanyloxy)-5-hydroxy-3,3',7-trimethoxyflavon,
4'-(L-Glutaminyloxy)-5-hydroxy-3,3',7-trimethoxyflavon,
4'-(L-α-Glutamyloxy)-5-hydroxy-3,3',7-trimethoxyflavon.

4. Eine der nachfolgenden Verbindungen gemäss Anspruch 1:
4'-Amino-5,7-dihydroxy-3-methoxyflavon,
4'-Amino-5-hydroxy-3,7-dimethoxyflavon,
3-Ethoxy-4',5-dihydroxy-3',7-dimethoxyflavon und
4',5-Dihydroxy-3-isopropoxy-3',7-dimethoxyflavon.

5. Verfahren zur Herstellung von 3-Alkoxyflavonderivaten der allgemeinen Formel

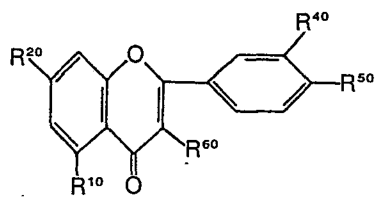 II

worin
$R^{10}$ Hydroxy, $C_{2-7}$-Alkanoyloxy, $C_{2-7}$-Alkoxycarbonyloxy oder Nicotinoyloxy;
$R^{20}$ Hydroxy oder $C_{1-4}$-Alkoxy;
$R^{40}$ Wasserstoff oder $C_{1-4}$-Alkoxy;
$R^{50}$ Hydroxy, $C_{2-7}$-Alkanoyloxy, L-Lysyloxy, L-Alanyloxy, L-Glutaminyloxy, α-Glutamyloxy, Glucosyloxy, Mannosyloxy, Galaktosyloxy, einen 1-Hydroxy-1,2-ethandicarbonsäurerest, einen 1,2-Dihydroxy-1,2-ethandicarbonsäurerest, Amino, Nicotinoyloxy oder $C_{2-7}$-Alkoxycarbonyloxy und
$R^{60}$ $C_{1-4}$-Alkoxy darstellen,
mit der Einschränkung, dass $R^{50}$ nicht Acetoxy darstellt, wenn $R^{10}$ Acetoxy ist, und dass $R^{60}$ nicht Methoxy darstellt, wenn $R^{50}$ Hydroxy ist,
dadurch gekennzeichnet, dass man

a) die Hydroxylgruppe in 4'-Stellung oder die Hydroxylgruppen in 1- und 4'-Stellung einer Verbindung der allgemeinen Formel

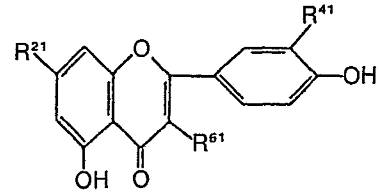 III

worin
$R^{21}$ $C_{1-4}$-Alkoxy; $R^{41}$ Wasserstoff oder $C_{1-4}$-Alkoxy und $R^{61}$ $C_{1-4}$-Alkoxy darstellen,
mit einem für eine Acylierung geeigneten reaktionsfähigen Derivat einer $C_{2-7}$-Alkancarbonsäure von L-Lysin, L-Alanin, L-Glutamin, α-Glutamat oder 1-Hydroxy-1,2-ethandicarbonsäure, oder 1,2-Dihydroxy-1,2-ethandicarbonsäure oder der Nicotinsäure umsetzt, oder

(b) die Hydroxylgruppe in 4'-Stellung einer Verbindung der Formel III mit einem für eine Glycosilierung geeigneten reaktionsfähigen Derivat eines entsprechenden Monosaccharids glycolysiert, oder

(c) ein 2',4',6'-Trihydroxy-2-alkoxyacetophenon mit einem Bis-(4-acetamidobenzoesäure)-anhydrid und einem Alkalimetallsalz der 4-Acetamidobenzoesäure behandelt und die erhaltene Verbindung mit einem Alkalimetallhydroxid zu einem 4'-Amino-5,7-dihydroxy-3-$C_{1-4}$-alkoxyflavon umsetzt, und gewünschtenfalls durch weitere Umsetzung mit einem Diazoalkan in die entsprechende 7-$C_{1-4}$-Alkoxyverbindung überführt.

6. Ein 3-Alkoxyflavonderivat der Formel

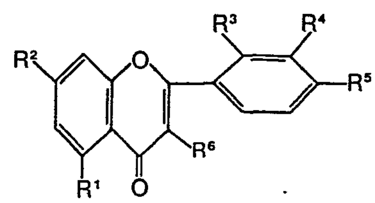 I

worin
$R^1$ Hydroxy, $C_{1-4}$-Alkoxy, $C_{2-7}$-Alkanoyloxy, $C_{2-7}$-Alkoxycarbonyloxy oder Nicotinoyloxy;
$R^2$ Hydroxy oder $C_{1-4}$-Alkoxy;
$R^3$ Wasserstoff oder $C_{1-4}$-Alkoxy;
$R^4$ Wasserstoff, Hydroxy oder $C_{1-4}$-Alkoxy;
$R^5$ Hydroxy, $C_{1-4}$-Alkoxy, $C_{2-7}$-Alkanoyloxy, L-Lysyloxy, L-Alanyloxy, L-Glutaminyloxy, α-Glutamyloxy, Glucosyloxy, Mannosyloxy, Galaktosyloxy, einen 1-Hydroxy-1,2-ethandicarbonsäurerest, einen 1,2-Dihydroxy-1,2-ethandicarbonsäurerest, Amino, Nicotinoyloxy oder $C_{2-7}$-Alkoxycarbonyloxy und
$R^6$ $C_{1-4}$-Alkoxy darstellen,
zur Verwendung als antiviraler Wirkstoff.

7. Ein antivirales Arzneimittel, das als aktiven Wirkstoff ein 3-Alkoxyflavonderivat der Formel

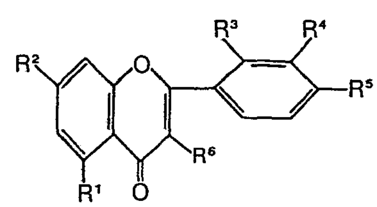 I

gemäss Anspruch 6,
worin
$R^1$ Hydroxy, $C_{1-4}$-Alkoxy, $C_{2-7}$-Alkanoyloxy, $C_{2-7}$-Alkoxycarbonyloxy oder Nicotinoyloxy;
$R^2$ Hydroxy oder $C_{1-4}$-Alkoxy;
$R^3$ Wasserstoff oder $C_{1-4}$-Alkoxy;
$R^4$ Wasserstoff, Hydroxy oder $C_{1-4}$-Alkoxy;
$R^5$ Hydroxy, $C_{1-4}$-Alkoxy, $C_{2-7}$-Alkanoyloxy, L-Lysyloxy, L-Alanyloxy, L-Glutaminyloxy,α-Glutamyloxy, Glucosyloxy, Mannosyloxy, Galaktosyloxy, einen 1-Hydroxy-1,2-ethandicarbonsäurerest, einen 1,2-Dihydroxy-1,2-ethandicarbonsäurerest, Amino-Nicotinoyloxy oder $C_{2-7}$-Alkoxycarbonyloxy und $R^6$ $C_{1-4}$-Alkoxy darstellen,
enthält.

**Claims**

1. 3-Alkoxyflavone derivatives of the general formula

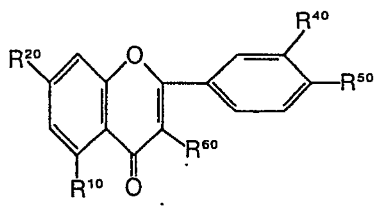
II

wherein
$R^{10}$ represents hydroxy, $C_{2-7}$-alkanoyloxy, $C_{2-7}$-alkoxycarbonyloxy or nicotinoyloxy;
$R^{20}$ represents hydroxy or $C_{1-4}$-alkoxy;
$R^{40}$ represents hydrogen or $C_{1-4}$-alkoxy;
$R^{50}$ represents hydroxy, $C_{2-7}$-alkanoyloxy, L-lysyloxy, L-alanyloxy, L-glutaminyloxy, α-glutamyloxy, glucosyloxy, mannosyloxy, galactosyloxy, a 1-hydroxy-1,2-ethanedicarboxylic acid residue, a 1,2-dihydroxy-1,2-ethanedicarboxylic acid residue, amino, nicotinoyloxy or $C_{2-7}$-alkoxycarbonyloxy and
$R^{60}$ represents $C_{1-4}$-alkoxy,
with the proviso that $R^{50}$ does not represent acetoxy when $R^{10}$ is acetoxy and that $R^{60}$ does not represent methoxy when $R^{50}$ is hydroxy.

2. One of the following compounds in accordance with claim 1:
4′-Acetoxy-5-hydroxy-3,3′,7-trimethoxyflavone,
4′-(3-carboxy-2,3-dihydroxypropionyloxy)-5-hydroxy-3,3′,7-trimethoxyflavone,
3,3′,7-trimethoxy-4′,5-bis-(nicotinoyloxy)-flavone,
4′,5-bis-(ethoxycarbonyloxy)-3,3′,7-trimethoxyflavone,
5-hydroxy-3,3′,7-trimethoxy-4′-(pivaloyloxy)-flavone,
5-(isobutyryloxy(-3,3′,7-trimethoxy-4′-(pivaloyloxy)-flavone,
3,3′,7-trimethoxy-4′,5-bis-(propionyloxy)-flavone.

3. One of the following compounds in accordance with claim 1:
5-Hydroxy-4′-(L-lysyloxy)-3,3′,7-trimethoxyflavone,
4′-(L-alanyloxy)-5-hydroxy-3,3′,7-trimethoxyflavone,
4′-(L-glutaminyloxy)-5-hydroxy-3,3′,7-trimethoxyflavone,
4′-(L-α-glutamyloxy)-5-hydroxy-3,3′,7-trimethoxyflavone.

4. One of the following compounds in accordance with claim 1:
4′-Amino-5,7-dihydroxy-3-methoxyflavone,
4′-amino-5-hydroxy-3,7-dimethoxyflavone,
3-ethoxy-4′,5-dihydroxy-3′,7-dimethoxyflavone and
4′,5-dihydroxy-3-isopropoxy-3′,7-di-dimethoxyflavone.

5. A process for the manufacture of 3-alkoxyflavone derivatives of the general formula

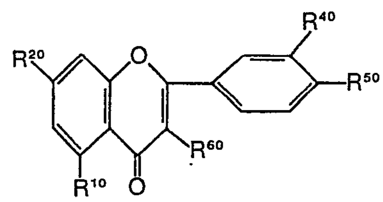
II

in accordance with claim 1,
wherein
$R^{10}$ represents hydroxy, $C_{2-7}$-alkanoyloxy, $C_{2-7}$-alkoxycarbonyloxy or nicotinoyloxy;
$R^{20}$ represents hydroxy or $C_{1-4}$-alkoxy;
$R^{40}$ represents hydrogen or $C_{1-4}$-alkoxy;
$R^{50}$ represents hydroxy, $C_{2-7}$-alkanoyloxy, L-lysyloxy, L-alanyloxy, L-glutaminyloxy, α-glutamyloxy, glucosyloxy, mannosyloxy, galactosyloxy, a 1-hydroxy-1,2-ethanedicarboxylic acid residue, a 1,2-dihydroxy-1,2-ethanedicarboxylic acid residue, amino, nicotinoyloxy or $C_{2-7}$-alkoxycarbonyloxy and
$R^{60}$ represents $C_{1-4}$-alkoxy,
with the proviso that $R^{50}$ does not represent acetoxy when $R^{10}$ is acetoxy and that $R^{60}$ does not represent methoxy when $R^{50}$ is hydroxy,
characterized by
a) reacting the hydroxyl group in the 4′-position or the hydroxyl groupy in the 1- and 4′-position of a compound of the general formula

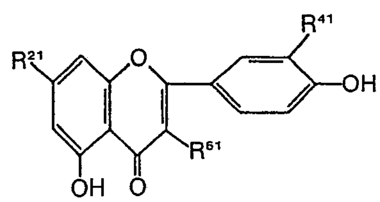
III

wherein
$R^{21}$ represents $C_{1-4}$-alkoxy;
$R^{41}$ represents hydrogen or $C_{1-4}$-alkoxy and
$R^{61}$ represents $C_{1-4}$-alkoxy,
with a reactive derivative of a $C_{2-7}$-alkanecarboxylic acid, of L-lysine, L-alanine, L-glutamine, α-glutamate, of 1-hydroxy-1,2-ethanedicarboxylic acid, of 1,2-dihydroxy-1,2-ethanedicarboxylic acid or of nicotinic acid which is suitable for an acylation, or

(b) glycosylating the hydroxyl group in the 4′-position of a compound of formula III with a reactive derivative of a corresponding monosaccharide which is suitable for a glycosylation, or

(c) treating a 2',4',6'-trihydroxy-2-alkoxy-acetophenone with a bis-(4-acetamidobenzoic acid) anhydride and an alkali metal salt of 4-acetamidobenzoic acid and reacting the compound obtained with an alkali metal hydroxide to give a 4'-amino-5,7-dihydroxy-3-$C_{1-4}$-alkoxyflavone, and, if desired, converting said compound into the corresponding 7-$C_{1-4}$-alkoxy compound by further reacting with a diazoalkane.

6. A 3-alkoxyflavone derivative of the formula

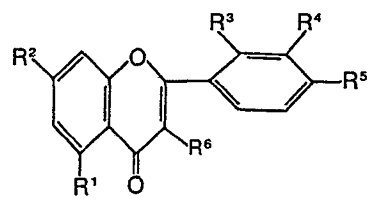

I

wherein

$R^1$ represents hydroxy, $C_{1-4}$-alkoxy, $C_{2-7}$-alkanoyloxy, $C_{2-7}$-alkoxycarbonyloxy or nicotinoyloxy;

$R^2$ represents hydroxy or $C_{1-4}$-alkoxy;

$R^3$ represents hydrogen or $C_{1-4}$-alkoxy;

$R^4$ represents hydrogen, hydroxy or $C_{1-4}$-alkoxy;

$R^5$ represents hydroxy, $C_{1-4}$-alkoxy, $C_{2-7}$-alkanoyloxy, L-lysyloxy, L-alanyloxy, L-glutaminyloxy, α-glutamyloxy, glucosyloxy, mannosyloxy, galactosyloxy, a 1-hydroxy-1,2-ethanedicarboxylic acid residue, a 1,2-dihydroxy-1,2-ethanedicarboxylic acid residue, amino, nicotinoyloxy or $C_{2-7}$-alkoxycarbonyloxy and

$R^6$ represents $C_{1-4}$-alkoxy,

for use as an antiviral active substance.

7. An antiviral medicament which contains as the active substance a 3-alkoxyflavone derivative of the formula

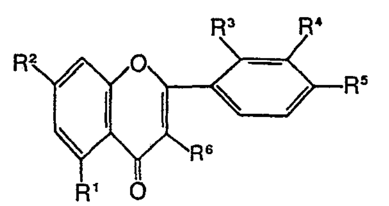

I

in accordance with claim 6,

wherein

$R^1$ represents hydroxy, $C_{1-4}$-alkoxy, $C_{2-7}$-alkanoyloxy, $C_{2-7}$-alkoxycarbonyloxy or nicotinoyloxy;

$R^2$ represents hydroxy or $C_{1-4}$-alkoxy;

$R^3$ represents hydrogen or $C_{1-4}$-alkoxy;

$R^4$ represents hydrogen, hydroxy or $C_{1-4}$-alkoxy;

$R^5$ represents hydroxy, $C_{1-4}$-alkoxy, $C_{2-7}$-alkanoyloxy, L-lysyloxy, L-alanyloxy, L-glutaminyloxy, α-glutamyloxy, glucosyloxy, mannosyloxy, galactosyloxy, a 1-hydroxy-1,2-ethanedicarboxylic acid residue, a 1,2-dihydroxy-1,2-ethanedicarboxylic acid residue, amino, nicotinoyloxy or $C_{2-7}$-alkoxycarbonyloxy and

$R^6$ represents $C_{1-4}$-alkoxy.

**Revendications**

1. Dérivés de 3-alcoxyflavone de formule générale

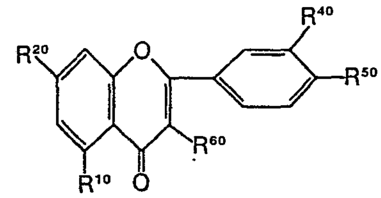

II

où

$R^{10}$ représente un hydroxy, un alcanoyloxy en $C_2$ à $C_7$, un alcoxy en $C_2$ à $C_7$-carbonyloxy ou un nicotinoyloxy,

$R^{20}$ représente un hydroxy ou un alcoxy en $C_1$ à $C_4$;

$R^{40}$ représente un hydrogène ou un alcoxy en $C_1$ à $C_4$;

$R^{50}$ représente un hydroxy, un alcanoyloxy en $C_2$ à $C_7$, un L-lysyloxy, un L-alanyloxy, un L-glutaminyloxy, un α-glutamyloxy, un glucosyloxy, un mannosyloxy, un galactosyloxy, un radical acide 1-hydroxy-1,2-éthanedicarboxylique, un radical acide 1,2-dihydroxy-1,2-éthanedicarboxylique, un amino, un nicotinoyloxy ou un alcoxy en $C_2$ à $C_7$-carbonyloxy et

$R^{60}$ représente un alcoxy en $C_1$ à $C_4$,

avec cette limitation que $R^{50}$ ne représente pas un acétoxy lorsque $R^{10}$ est un acétoxy, et que $R^{60}$ ne représente pas un méthoxy lorsque $R^{50}$ est un hydroxy.

2. L'un des composés suivants selon la revendication 1:

4'-acétoxy-5-hydroxy-3,3',7-triméthoxyflavone,
4'-(3-carboxy-2,3-dihydroxypropionyloxy)-5-hydroxy-3,3',7-triméthoxyflavone,
3,3',7-triméthoxy-4',5-bis-(nicotinoyloxy)-flavone,
4',5-bis-(éthoxycarbonyloxy)-3,3',7-triméthoxyflavone,
5-hydroxy-3,3',7-triméthoxy-4'-(pivaloyloxy)-flavone,
5-(isobutyryloxy)-3,3',7-triméthoxy-4'-(pivaloyloxy)-flavone.
3,3',7-triméthoxy-4',5-bis-(propionyloxy)-flavone.

3. L'un des composés suivants selon la revendication 1:

5-hydroxy-4'-(L-lysyloxy)-3,3',7-triméthoxyflavone,
4'-(L-alanyloxy)-5-hydroxy-3,3',7-triméthoxyflavone,
4'-(L-glutaminyloxy)-5-hydroxy-3,3',7-triméthoxyflavone,
4'-(L-α-glutamyloxy)-5-hydroxy-3,3',7-triméthoxyflavone.

4. L'un des composés suivants selon la revendication 1:

4'-amino-5,7-dihydroxy-3-méthoxyflavone,
4'-amino-5-hydroxy-3,7-diméthoxyflavone,
3-éthoxy-4',5-dihydroxy-3',7-diméthoxyflavone et
4',5-dihydroxy-3-isopropoxy-3',7-diméthoxyflavone.

5. Procédé de préparation de dérivés de 3-alcoxyflavone de formule générale

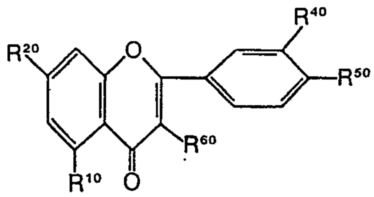

II

selon la revendication 1,

où

$R^{10}$ représente un hydroxy, un alcanoyloxy en $C_2$ à $C_7$, un alcoxy en $C_2$ á $C_7$-carbonyloxy ou un nicotinoyloxy,

$R^{20}$ représente un hydroxy ou un alcoxy en $C_1$ à $C_4$;

$R^{40}$ représente un hydrogène ou un alcoxy en $C_1$ à $C_4$;

$R^{50}$ représente un hydroxy, un alcanoyloxy en $C_2$ à $C_7$, un L-lysyloxy, un L-alanyloxy, un L-glutaminyloxy, un α-glutamyloxy, un glucosyloxy, un mannosyloxy, un galactosyloxy, un radical acide 1-hydroxy-1,2-éthane-dicarboxylique, un radical acide 1,2-dihydroxy-1,2-éthanedicarboxylique, un amino, un nicotinoyloxy ou un alcoxy en $C_2$ à $C_7$-carbonyloxy et

$R^{60}$ représente un alcoxy en $C_1$ à $C_4$, avec cette limitation que $R^{50}$ ne représente pas un acétoxy lorsque $R^{10}$ est un acétoxy, et que $R^{60}$ ne représente pas un méthoxy lorsque $R^{50}$ est un hydroxy, caractérisé en ce que

a) on fait réagir le groupe hydroxyle en position 4′ ou les groupes hydroxyles en position 1 et 4′ d'un composé de formule générale

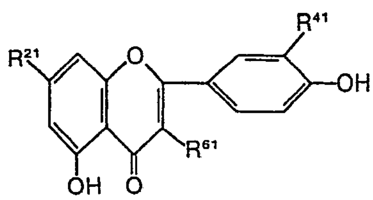

III

où

$R^{21}$ représente un alcoxy en $C_1$ à $C_4$;

$R^{41}$ représente un hydrogène ou un alcoxy en $C_1$ à $C_4$ et

$R^{61}$ représente un alcoxy en $C_1$ à $C_4$, avec un dérivé réactif, approprié pour une acylation, d'un acide alcane en $C_2$ à $C_7$-carboxylique de L-lysine, L-alanine, L-glutamine, α-glutamate de l'acide 1-hydroxy-1,2-éthanedicarboxylique, de l'acide 1,2-dihydroxy-1,2-éthanedicarboxylique ou de l'acide nicotinique, ou

b) on glycosyle le groupe hydroxyle en position 4′ d'un composé de formule III avec un dérivé réactif, approprié pour une glycosylation, d'un monosaccharide correspondant, ou

c) On traite une 2′,4′,6′-trihydroxy-2-alcoxyacétophénone avec un anhydride d'acide bis-(4-acétamidobenzoïque) et un sel de métal alcalin de l'acide 4-acétamidobenzoïque et on fait réagir le composé obtenu avec un hydroxyde de métal alcalin pour donner une 4′-amino-5,7-dihydroxy-3-(alcoxy en $C_1$ à $C_4$)-flavone et si on le désire on transforme par une réaction ultérieure avec un diazoalcane pour donner le composé 7-(alcoxy en $C_1$ à $C_4$) correspondant.

6. Dérivé de 3-alcoxyflavone de formule

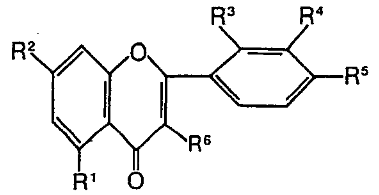

I

où

$R^1$ représente un hydroxy, un alcoxy en $C_1$ à $C_4$, un alcanoyloxy en $C_2$ à $C_7$, un alcoxy en $C_2$ à $C_7$-carbonyloxy ou un nicotinoyloxy;

$R^2$ représente un hydroxy ou un alcoxy en $C_1$ à $C_4$;

$R^3$ représente un hydrogène ou un alcoxy en $C_1$ à $C_4$;

$R^4$ représente un hydrogène, un hydroxy ou un alcoxy en $C_1$ à $C_4$;

$R^5$ représente un hydroxy, un alcoxy en $C_1$ à $C_4$, un alcanoyloxy en $C_2$ à $C_7$, un L-lysyloxy, un L-alanyloxy, un L-glutaminyloxy, un α-glutamyloxy, un glucosyloxy, un mannosyloxy, un galactosyloxy, un radical acide 1-hydroxy-1,2-éthanedicarboxylique, un radical acide 1,2-dihydroxy-1,2-éthanedicarboxylique, un amino, un nicotinoyloxy ou un alcoxy en $C_2$ à $C_7$ et

$R^6$ représente un alcoxy en $C_1$ à $C_4$, aux fins d'application comme substance active antivirale.

7. Médicament antiviral contenant comme substance active un dérivé de 3-alcoxyflavone de formule

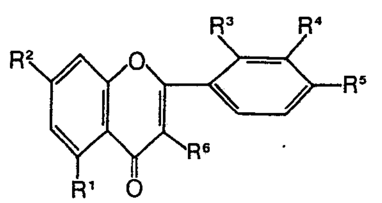

I

selon la formule 6, où

$R^1$ représente un hydroxy, un alcoxy en $C_1$ à $C_4$, un alcanoyloxy en $C_2$ à $C_7$, un alcoxy en $C_2$ à $C_7$-carbonyloxy ou un nicotinoyloxy;

$R^2$ représente un hydroxy ou un alcoxy en $C_1$ à $C_4$;

$R^3$ représente un hydrogène ou un alcoxy en $C_1$ à $C_4$;

$R^4$ représente un hydrogène, un hydroxy ou un alcoxy en $C_1$ à $C_4$;

$R^5$ représente un hydroxy, un alcoxy en $C_1$ à $C_4$, un alcanoyloxy en $C_2$ à $C_7$, un L-lysyloxy, un L-alanyloxy, un L-glutaminyloxy, un α-glutamyloxy, un glucosyloxy, un mannosyloxy, un galactosyloxy, un radical acide 1-hydroxy-1,2-éthanedicarboxylique, un radical acide 1,2-dihydroxy-1,2-éthanedicarboxylique, un amino, un nicotinoyloxy ou un alcoxy en $C_2$ à $C_7$-carbonyloxy et

$R^6$ représente un alcoxy en $C_1$ à $C_4$.

Fig. 1. Hemmung der viralen Replikation durch 4',5-Dihydroxy-3,3',7-trimethoxyflavon

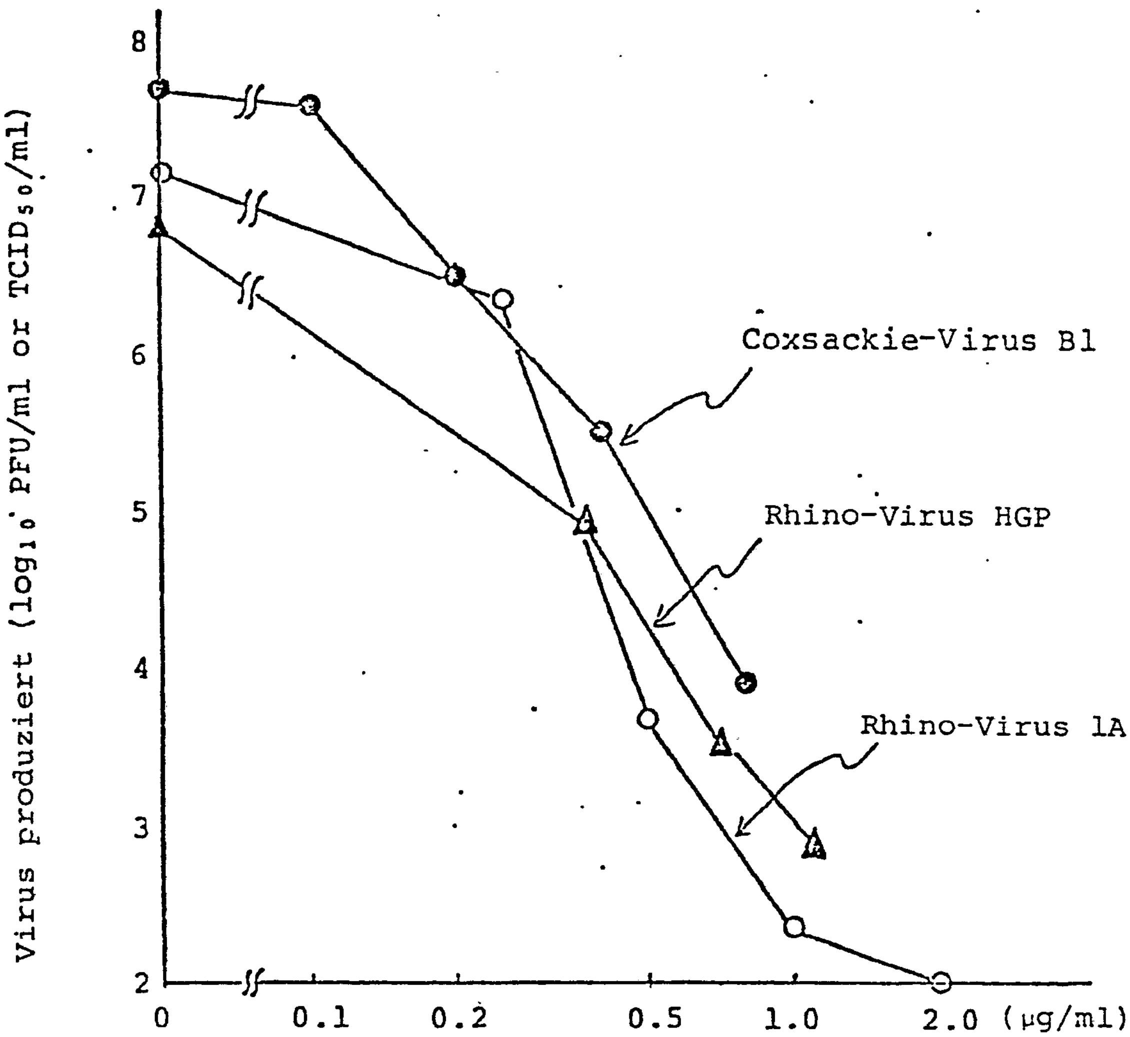


4',5-Dihydroxy-3,3',7-trimethoxyflavon